(19) Europäisches Patentamt
European Patent Office
Office européen des brevets

(11) Publication number: 0 005 986 A1

# (12) EUROPEAN PATENT APPLICATION

(21) Application number: 79300976.2

(22) Date of filing: 29.05.79

(51) Int. Cl.[2]: C 07 D 239/42
C 07 D 251/12, C 07 D 409/12
A 01 N 9/14, A 01 N 9/22
C 07 C 157/14

(30) Priority: 30.05.78 US 910966
16.04.79 US 29821

(43) Date of publication of application:
12.12.79 Bulletin 79/25

(84) Designated Contracting States:
BE DE FR GB IT LU NL SE

(71) Applicant: E.I. du Pont de Nemours and Company
1007 Market Street
Wilmington, Del. 19898(US)

(72) Inventor: Grantham, Gary Douglas
5505 Limeric Circle Apartment 12
Wilmington Delaware 19808(US)

(72) Inventor: Levitt, George
3218 Romilly Road Cardiff
Wilmington Delaware 19810(US)

(74) Representative: Hildyard, Edward Martin et al,
Frank B. Dehn & Co. Imperial House 15-19 Kingsway
London WC2B 6UZ(GB)

(54) Herbicidal carbamimidothioates, preparation and use thereof and compositions containing said compounds.

(57) Compounds of the formula

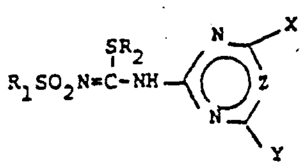

and their agriculturally suitable salts, wherein:
$R_1$ is thienyl or optionally substituted phenyl,
$R_2$ is selected from various organic groups,
X is $CH_3$, $CH_3O$, $CH_3CH_2O$ or $CH_3OCH_2$,
Y is $CH_3$ or $CH_3O$, and
Z is CH or N,
can be employed as general or selective herbicides and as plant growth modifiers. The compounds can be formulated for use in conventional manner. They can be prepared by alkylation of an appropriately substituted carbamimidothioic acid salt.

EP 0 005 986 A1

TITLE MODIFIED
see front page





Agricultural carbamimidothioates and preparation and use thereof

This invention relates to alkyl N-heterocyclic-N'-(arylsulfonyl)carbamimidothioates having agricultural activity. It relates also to such compounds wherein the aryl radical has been replaced by a thienyl radical.

In our United States Patent Specification No. 4,127,405 we have disclosed, inter alia, compounds of the following formula as herbicides:

$$R_1-SO_2-NH-\overset{S}{\overset{\|}{C}}-NH-R .$$

In the former R is:

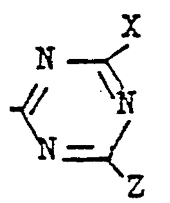

and in the latter, R is

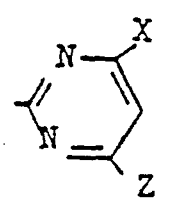

In each of those applications $R_1$ may be

U.S. Patent 3,823,007 discloses isourea salts as herbicides:

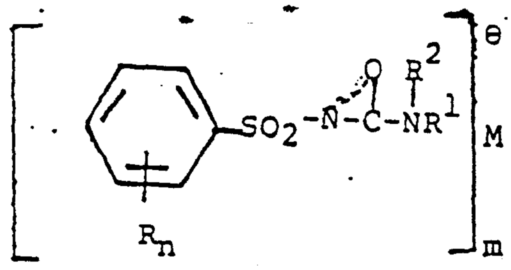

wherein R is chloro, bromo, iodo, cyano, alkyl, alkoxy, nitro, amino or

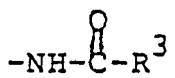 ;

$R^3$ is hydrogen or alkyl;

n is the integer one to three;

m is the integer one or two;

M is of alkali metal, alkaline earth metal or ammonium;

$R^1$ and $R^2$ are each independently hydrogen, alkyl, alkoxy, alkynyl, phenyl, substituted phenyl having a maximum of three substituents said substituents being alkyl bromine, chlorine, alkoxy, phenoxy, mono and dihalogenated phenoxy said halogen being chlorine or bromine, or the group

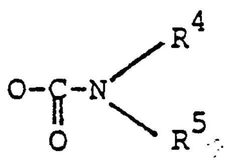 ; and

$R^4$ and $R^5$ are each independently hydrogen or alkyl.

U.S. Patent 3,714,209 discloses the isopropylidineaminoethanol salt of p-nitrobenzenesulfonylisourea as a herbicide:

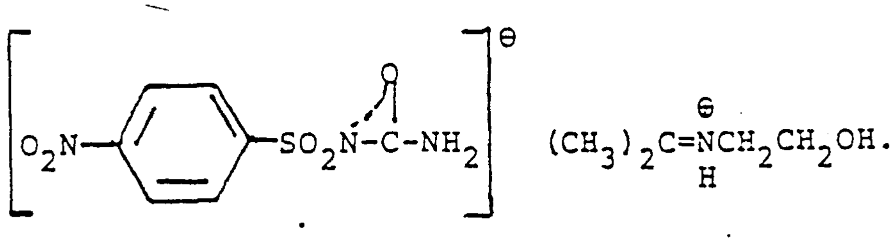

$R_2$ is $C_1$-$C_{10}$ alkyl; $CH_2CH_2OCH_3$; $CH_2CH_2OCH_2CH_3$; $CH_2CH_2CH_2OCH_3$; $CH_2A$, 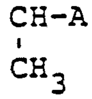 where A is $CO_2$-$C_1$-$C_4$ alkyl; 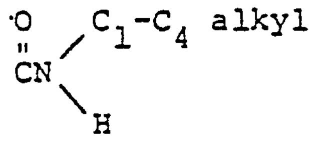 ; 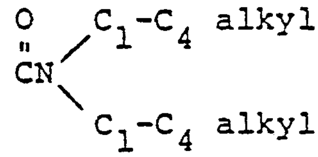 ; 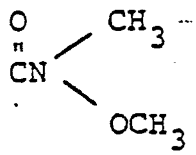 ; 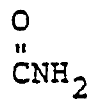; phenyl; phenyl substituted with $CH_3$ or chlorine; CN; $C_2$-$C_4$ alkenyl; $C_2$-$C_4$ alkynyl;

$R_3$ is H, Cl, Br, $CH_3$, $CH_3O$, $NO_2$, $CF_3$, $CH_3S(O)_n$, $COOR_5$, or $CONR_6R_7$;

$R_4$ is H, F, Cl, Br, $CH_3$, $CH_3O$, $NO_2$, $CF_3$, or $CH_3S(O)_n$; wherein n = 0, 1 or 2;

$R_5$ is $C_1$-$C_4$ alkyl or $C_3$-$C_4$ alkenyl;

$R_6$ is H, $CH_3O$ or $C_1$-$C_3$ alkyl;

$R_7$ is H or $C_1$-$C_3$ alkyl;

X is $CH_3$, $CH_3O$, $CH_3CH_2O$ or $CH_3OCH_2$;

Y is $CH_3$ or $CH_3O$; and

Z is CH or N;

provided that $R_3$ and $R_4$ differ from one another when either of them is $NO_2$ or $CF_3$; and

that when $R_6$ is $CH_3O$, $R_7$ is $CH_3$.

Preferred for their high activity and/or favorable cost are those compounds of Formula I wherein independently or in any combination

A) The generic scope I where $R_1$ is 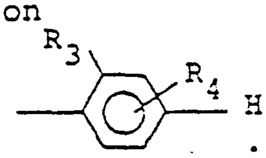.

B) Compounds of A where $R_3$ is Cl, Br, $CH_3$, $CH_3O$, $NO_2$ or $CO_2R_5$ and $R_4$ is H or Cl.

Specifically preferred for their outstanding activity and/or highly favorable cost are (melting points in °C):

methyl N'-(2,5-dimethoxyphenylsulfonyl)-N-(4,6-dimethoxypyrimidin-2-yl)carbamimidothioate, m.p. 207-208;

methyl N'-(2-chlorophenylsulfonyl)-N-(4,6-dimethoxypyrimidin-2-yl)carbamimidothioate, m.p. 169-171;

methyl N'-(2-chlorophenylsulfonyl)-N-(4-methoxy-6-methylpyrimidin-2-yl)carbamimidothioate, m.p. 160-162;

methyl N'-(2-chlorophenylsulfonyl)-N-(4,6-dimethoxy-1,3,5-triazine-2-yl)carbamimidothioate.

methyl N'-(2-chlorophenylsulfonyl)-N-(4-methoxy-6-methyl-1,3,5-triazin-2-yl)carbamimidothioate, m.p. 148-150°;

methyl 2-[(2-chlorophenylsulfonylimino)(4-methoxy-6-methylpyrimidin-2-ylamino)methylthio]-propionate, m.p. 166-168°.

Synthesis

As shown in Equation 1, the compounds of Formula I can be prepared by reacting an appropriately substituted carbamimidothioic acid salt of Formula II with an alkylating agent of Formula III:

Equation I:

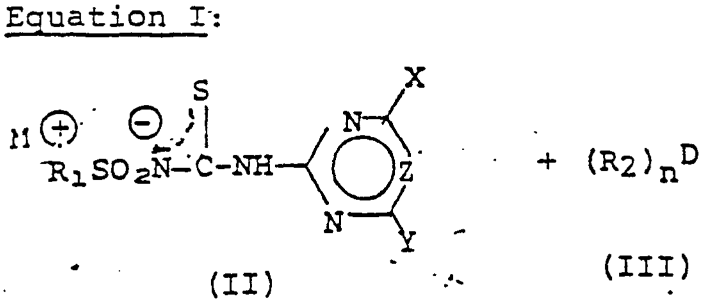

(II) (III)

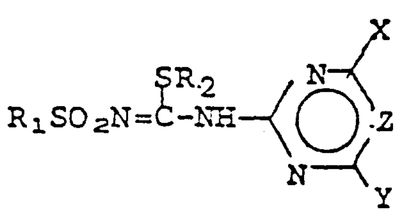

(I)

add the isothiocyanate to a stirred suspension of the aminoheterocycle. Since such isothiocyanates usually are liquids, their addition is more easily controlled. Catalysts, such as 1,4-diazabicyclo[2.2.2]octane or dibutyltindilaurate, may be used.

In some cases, sulfonylthioureas of Formula IV are more conveniently prepared, as shown in Equation 3, by reacting an appropriately substituted sulfonamide of Formula VII with the appropriate 2-isothiocyanatoheterocycle of Formula VIII; $R_1$, X, Y and Z being as previously defined:

Equation 3

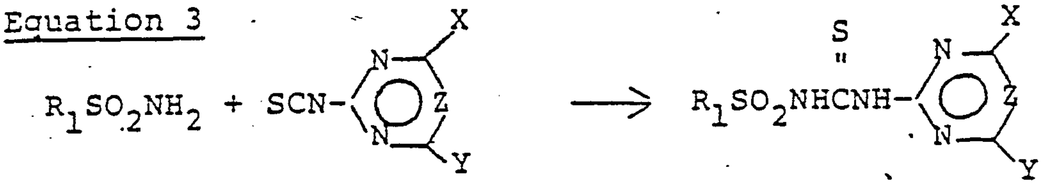

(VII) (VIII) (IV)

The preparation of 2-isothiocyanatoheterocycles VIII is taught in Japanese patent Kokai 51-143686.

The preparation of the sulfonyl thioureas IV and reactants therefor is described in said United Patent Specification No. 4,127,405, the contents of which in that connection are incorporated herein by reference.

The compounds of Formula I may also be prepared as shown in Equation 4. This is the preferred procedure for Z = N.

Equation 4

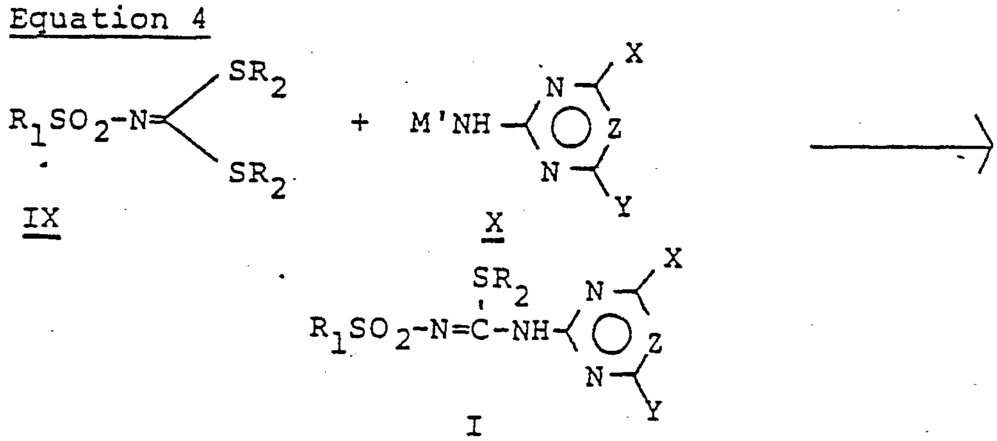

IX X

I

reaction mixture was refluxed for 0.5 hour, whereupon 5.5 g of methyl iodide in 10 ml of anhydrous tetrahydrofuran was added. After refluxing for an additional 4 hours the reaction mixture was cooled, solvent was removed under vacuum, and the residue recrystallized from acetonitrile to afford 6.5 g of methyl N'-(2-chlorophenylsulfonyl)-N-(4,6-dimethoxypyrimidin-2-yl)carbamimidothioate, m.p. 169-171°C.

## EXAMPLE 3

Methyl N'-(2-chlorophenylsulfonyl)-N-(4-methoxy-6-methylpyrimidin-2-yl)carbamimidothioate

To a suspension of 11.2 g of N-[4-methoxy-6-methylpyrimidin-2-yl)aminothioxomethyl]-2-chlorobenzenesulfonamide in 150 ml of anhydrous tetrahydrofuran was added 13.0 ml of 3M $NaOCH_3$/MeOH solution. The reaction mixture was refluxed for 0.5 hour, whereupon 5.5 g of methyl iodide in 10 ml of anhydrous tetrahydrofuran was added. After refluxing for an additional 4 hours, the reaction mixture was cooled, solvent was removed under vacuum and the residue was recrystallized from acetonitrile to afford 8.6 g of methyl N'(2-chlorophenylsulfonyl)-N-(4-methoxy-6-methylpyrimidin-2-yl)carbamimidothioate, m.p. 160-162°C.

## EXAMPLE 4

Methyl N'(2,5-dimethoxyphenylsulfonyl)-N-(4-methoxy-6-methyl-1,3,5-triazine-2-yl)carbonimidothioate

1.4 grams of 2-amino-4-methoxy-6-methyltriazine was dissolved in a mixture of 20 ml of tetrahydrofuran and 60 ml of dimethylformamide. 0.48 grams of sodium hydride (50% mineral oil dispersion) was added. The reaction was stirred until hydrogen evolution ceased. 2.75 grams of N(2,5-dimethoxyphenylsulfonyl)-carbonimidodithoic acid, S,S-dimethyl ester was added all at once. The reaction mixture was stirred 24 hours at ambient temperature. The mixture was poured into 300 ml of water and acidified to a pH of 5 with 20% hydrochloric acid. The mixture was filtered. The crude solid obtained was crystallized from ethyl acetate to yield 1.1 grams of product with m.p. 168-169°.

TABLE I

| $R_2$ | $R_3$ | $R_4$ | X | Y | m.p. |
|---|---|---|---|---|---|
| $CH_3CH_2$ | $NO_2$ | H | $CH_3$ | $CH_3$ | |
| $CH_3(CH_2)_2$ | $NO_2$ | H | $CH_3O$ | $CH_3$ | |
| $CH_3CH_2(CH_3)CH$ | Cl | 5-Cl | $CH_3O$ | $CH_3$ | |
| $(CH_3)_2CHCH_2$ | $NO_2$ | H | $CH_3O$ | $CH_3$ | |
| $CH_3$ | $CH_3SO_2$ | 5-$CH_3SO_2$ | $CH_3O$ | $CH_3$ | |
| $CH_3$ | $CH_3SO$ | 5-$CH_3SO$ | $CH_2CH_3O$ | $CH_3$ | |
| $(CH_3)_2CH$ | Cl | H | $CH_3O$ | $CH_3$ | 255-258 (dec.) |
| $CH_3(CH_2)_3$ | Cl | H | $CH_3O$ | $CH_3O$ | 233-235 (dec.) |
| $(CH_3)_2CHCH_2$ | Cl | H | $CH_3O$ | $CH_3$ | 106-107° |
| $CH_3(CH_2)_3$ | Cl | H | $CH_3O$ | $CH_3$ | |
| $(CH_3)_2CH$ | $CH_3O$ | 5-$CH_3O$ | $CH_3O$ | $CH_3$ | |
| $CH_3CH_2$ | $CH_3O$ | 5-$CH_3O$ | $CH_3O$ | $CH_3O$ | 195-198 (dec.) |
| $CH_3(CH_2)_5$ | Cl | H | $CH_3O$ | $CH_3$ | 118-120° |
| $CH_3(CH_2)_3CH$<br>$CH_3$ | $CH_3O$ | 5-$CH_3O$ | $CH_3$ | $CH_3O$ | |
| $CH_3$ | $COOCH_3$ | H | $CH_3$ | $CH_3O$ | |
| $CH_3$ | $CON(CH_3)_2$ | H | $CH_3$ | $CH_3O$ | |
| $CH_3(CH_2)_3$ | $CH_3$ | H | $CH_3$ | $CH_3O$ | 115-116° |
| $(CH_3)_2CH$ | $CH_3$ | H | $CH_3$ | $CH_3O$ | 90-93.5° |
| $CH_3(CH_2)_4$ | H | H | $CH_3$ | $CH_3O$ | 102-106° |
| $(CH_3)_2CH$ | Cl | H | $CH_3$ | $CH_3$ | 113-122° |
| $(CH_3)_2CHCH_2$ | Cl | H | $CH_3$ | $CH_3$ | 108-112° |
| $CH_3CH_2CH$<br>$CH_3$ | Cl | H | $CH_3$ | $CH_3$ | 104-110° |
| $CH_3$ | Cl | H | $CH_3$ | $CH_3O$ | 160-162° |
| $CH_3$ | Cl | H | $CH_3O$ | $CH_3O$ | 169-171° |
| n-$C_7H_{15}$ | Cl | H | $CH_3$ | $CH_3$ | 114-115° |
| n-$C_{10}H_{21}$ | Cl | H | $CH_3$ | $CH_3O$ | 82- 87° |
| $CH(CH_2)_7CH_3$<br>$CH_3$ | Cl | H | $CH_3$ | $CH_3O$ | |
| $CH_2CH_2OCH_3$ | Cl | H | $CH_3$ | $CH_3$ | 137-139° |

TABLE I

| $R_2$ | $R_3$ | $R_4$ | X | Y | m.p. (°C) |
|---|---|---|---|---|---|
| $CH_2C\equiv C-CH_3$ | $CO_2CH_3$ | H | $CH_3$ | $CH_3$ | |
| $-CH(CH_3)-CH=C(CH_3)_2$ | $CO_2CH_3$ | H | $CH_3$ | $CH_3$ | |
| $-CH_2C\equiv C-C_2H_5$ | $CO_2CH_3$ | H | $CH_3$ | $CH_3$ | |
| $-CH_2CH_2CH(CH_3)_2$ | Cl | H | $CH_3$ | $CH_3$ | 93-105° |
| $-CH(CH_3)CH_2CH_2CH_3$ | Cl | H | $CH_3$ | $CH_3$ | 110-113° |
| $-(CH_2)_5CH_3$ | Cl | H | $CH_3O$ | $CH_3O$ | 112-114° |
| $-(CH_2)_7CH_3$ | Cl | H | $CH_3O$ | $CH_3O$ | 96- 98° |

TABLE II

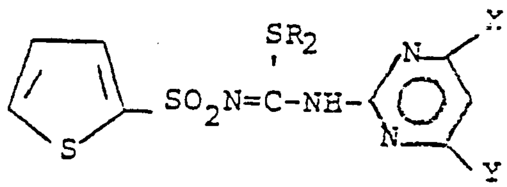

| $R_2$ | X | Y | m.p. |
|---|---|---|---|
| $CH_3$ | $CH_3O$ | $CH_3O$ | |
| $CH_3CH_2$ | $CH_3$ | $CH_3O$ | |
| $CH_3(CH_2)_2$ | $CH_3$ | $CH_3O$ | |
| $CH_3$ | $CH_3CH_2O$ | $CH_3O$ | |
| $CH_3$ | $CH_3CH_2O$ | $CH_3$ | |
| $CH_3$ | $CH_3$ | $CH_3$ | |
| $(CH_3)_2CH$ | $CH_3OCH_2$ | $CH_3$ | |
| $(CH_3)_2CHCH_2$ | $CH_3O$ | $CH_3O$ | |
| $CH_3CH_2(CH_3)CH$ | $CH_3O$ | $CH_3$ | |
| $CH_3(CH_2)_5$ | $CH_3O$ | $CH_3$ | |
| $CH_3(CH_2)_3$ | $CH_3O$ | $CH_3$ | 112-113.5° |

Using the procedures of Examples 1-3 with equivalent amounts of appropriate N-(triazinylamino-thioxomethyl)benzenesulfonamides (or thiophenesulfonamides), metal compounds and alkylating agents, the compounds of Tables III and IV can be prepared.

0005986

TABLE III

| $R_2$ | $R_3$ | $R_4$ | X | Y | m.p. (°C) |
|---|---|---|---|---|---|
| $CH_3$ | H | H | $CH_3CH_2O$ | $CH_3$ | |
| $CH_3$ | Cl | H | $CH_3O$ | $CH_3$ | 148-150° |
| $CH_3CH_2$ | Br | H | $CH_3$ | $CH_3$ | |
| $CH_3$ | F | H | $CH_3OCH_2$ | $CH_3$ | |
| $CH_3CH_2$ | $CH_3$ | H | $CH_3CH_2O$ | $CH_3$ | |
| $CH_3$ | $CF_3$ | H | $CH_3$ | $CH_3$ | |
| $CH_3$ | $NO_2$ | H | $CH_3CH_2O$ | $CH_3$ | |
| $CH_3$ | $CH_3O$ | H | $CH_3$ | $CH_3$ | |
| $CH_3$ | Cl | 5-Cl | $CH_3CH_2O$ | $CH_3$ | |
| $CH_3$ | Cl | 6-Cl | $CH_3$ | $CH_3$ | |
| $CH_3$ | Br | 5-Br | $CH_3OCH_2$ | $CH_3$ | |
| $CH_3$ | $CH_3O$ | 5-$CH_3O$ | $CH_3O$ | $CH_3$ | 168-169° |
| $CH_3$ | $CH_3$ | 5-$CH_3$ | $CH_3O$ | $CH_3$ | |
| $CH_3$ | Cl | 3-Cl | $CH_3$ | $CH_3$ | |
| $CH_3$ | $CH_3$ | 5-$NO_2$ | $CH_3$ | $CH_3$ | |
| $CH_3$ | $CH_3O$ | 5-Cl | $CH_3O$ | $CH_3$ | |
| $CH_3$ | Cl | 5-$NO_2$ | $CH_3O$ | $CH_3$ | |
| $CH_3$ | Cl | 5-F | $CH_3$ | $CH_3$ | |
| $CH_3$ | H | 3-$CH_3$ | $CH_3CH_2O$ | $CH_3$ | |
| $CH_3$ | H | 3-Br | $CH_3CH_2O$ | $CH_3$ | |
| $CH_3CH_2$ | H | H | $CH_3OCH_2$ | $CH_3$ | |
| $CH_3CH_2$ | Cl | H | $CH_3$ | $CH_3$ | |
| $CH_3$ | Br | H | $CH_3$ | $CH_3$ | |
| $(CH_3)_2CH$ | F | H | $CH_3$ | $CH_3$ | |
| $CH_3$ | $CH_3$ | H | $CH_3$ | $CH_3$ | |
| $CH_3$ | $CF_3$ | H | $CH_3CH_2O$ | $CH_3$ | |
| $CH_3CH_2$ | $NO_2$ | H | $CH_3CH_2O$ | $CH_3$ | |
| $CH_3CH_2$ | $CH_3O$ | H | $CH_3O$ | $CH_3$ | |
| $CH_3CH_2$ | Cl | 5-Cl | $CH_3$ | $CH_3$ | |
| $CH_3CH_2$ | Cl | 6-Cl | $CH_3O$ | $CH_3$ | |
| $CH_3$ | Br | 5-Br | $CH_3OCH_2$ | $CH_3$ | |
| $CH_3$ | $CH_3O$ | 5-$CH_3S$ | $CH_3O$ | $CH_3$ | |

(Table III continued)

| $R_2$ | $R_3$ | $R_4$ | X | Y | m.p. |
|---|---|---|---|---|---|
| 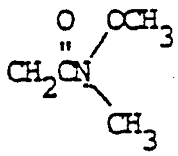 | Cl | H | $CH_3$ | $CH_3O$ | |
| $CH_2\overset{O}{\overset{\|}{C}}NH_2$ | Cl | H | $CH_3$ | $CH_3O$ | |

TABLE V

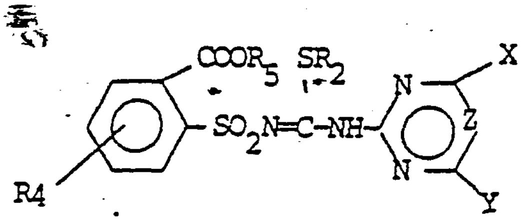

| $R_2$ | $R_4$ | $R_5$ | X | Y | Z | m.p. |
|---|---|---|---|---|---|---|
| $CH_3$ | H | $CH_3$ | $CH_3$ | $CH_3O$ | CH | |
| $CH_3$ | H | $CH_3$ | $CH_3O$ | $CH_3O$ | N | 170-172 |
| $CH_3(CH_2)_4$ | H | $CH_3$ | $CH_3CH_2O$ | $CH_3$ | CH | |
| $CH_3$ | 5-F | $CH_3$ | $CH_3OCH_2$ | $CH_3$ | CH | |
| $CH_3CH_2$ | 5-Cl | $CH_3$ | $CH_3O$ | $CH_3O$ | CH | |
| $(CH_3)_2CH$ | 5-Br | $CH_3$ | $CH_3$ | $CH_3O$ | CH | |
| $CH_3CH_2$ | 5-$NO_2$ | $CH_3$ | $CH_3$ | $CH_3O$ | CH | |
| $CH_3(CH_2)_3$ | 5-$CH_3O$ | $CH_3$ | $CH_3$ | $CH_3O$ | CH | |
| $CH_3(CH_2)_5$ | 5-$CH_3$ | $CH_3$ | $CH_3$ | $CH_3$ | CH | |
| $(CH_3)_2CHCH_2$ | 5-$CH_3O$ | $CH_3$ | $CH_3$ | $CH_3O$ | CH | |
| $CH_3(CH_2)_3\overset{CH_3}{CH}$ | 5-$CH_3$ | $CH_3$ | $CH_3CH_2O$ | $CH_3$ | CH | |
| $CH_3$ | 5-$CH_3S$ | $CH_3$ | $CH_3$ | $CH_3O$ | CH | |
| $CH_3$ | 5-Cl | $CH_2CH_3$ | $CH_3OCH_2$ | $CH_3$ | CH | |
| $CH_3$ | H | $CH_3CH_2\overset{CH_3}{CH}$ | $CH_3OCH_2$ | $CH_3O$ | CH | |
| $CH_3CH_2(CH_3)CH$ | H | $CH_3(CH_2)_3$ | $CH_3$ | $CH_3O$ | CH | |
| $CH_3CH_2CH_2$ | H | $CH_3CH_2CH_2$ | $CH_3CH_2O$ | $CH_3$ | CH | |
| $CH_3$ | H | $CH_3$ | $CH_3O$ | $CH_3$ | N | |
| $CH_3CH_2$ | 5-F | $CH_3$ | $CH_3OCH_2$ | $CH_3$ | N | |
| $CH_3$ | 5-$CH_3S$ | $CH_3$ | $CH_3O$ | $CH_3O$ | N | |
| $CH_3$ | 3-$CH_3$ | $CH_3CH_2$ | $CH_3$ | $CH_3O$ | N | |
| $(CH_3)_2CH$ | 6-Cl | $(CH_3)_2CH$ | $CH_3CH_2O$ | $CH_3$ | N | |
| $CH_3$ | 5-Br | $CH_3CH_2\overset{CH_3}{CH}$ | $CH_3O$ | $CH_3$ | N | |
| $CH_3$ | 5-$NO_2$ | $CH_3$ | $CH_3O$ | $CH_3$ | N | |
| $C_2H_5$ | H | $CH_3$ | $CH_3O$ | $CH_3O$ | N | syrup |
| $-\underset{CH_3}{CH}COOCH_3$ | H | $CH_3$ | $CH_3O$ | $CH_3O$ | N | 208-209 |
| $CH_3$ | H | $CH_2CH=CH_2$ | $CH_3O$ | $CH_3O$ | CH | |
| $CH_3$ | H | $CH(CH_3)CH=CH_2$ | $CH_3$ | $CH_3O$ | N | |

Formulations

Useful formulations of the compounds of Formula I can be prepared in conventional ways. They include dusts, granules, pellets, solutions, suspensions, emulsions, wettable powders, emulsifiable concentrates and the like. Many such formulations may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of a) about 0.1% to 20% surfactant(s) or b) about 1% to 99.9% solid or liquid diluent(s). More specifically, they will contain these ingredients in the approximate proportions set forth in Table VII.

TABLE VII

| | Weight Percent* | | |
|---|---|---|---|
| | Active Ingredient | Diluent(s) | Surfactant(s) |
| Wettable Powders | 20-90 | 0-74 | 1-10 |
| Oil Solutions, Suspensions, Emulsions (including Emulsifiable Concentrates) | 3-50 | 40-95 | 0-15 |
| Aqueous Suspensions | 10-50 | 40-84 | 1-20 |
| Dusts | 1-25 | 70-99 | 0-5 |
| Granules & Pellets | 0.1-95 | 5-99.9 | 0-15 |
| High Strength Compositions | 90-99 | 0-10 | 0-2 |

* Active Ingredient plus at least one of a surfactant or a diluent equals 100 weight percent.

Lower or higher levels of active ingredient can be present depending upon the intended use and the physical properties of the compound. Higher ratios of surfactant to active ingredient are sometimes desirable, and are achieved by incorporation into the formulation or by tank mixing.

19 and Examples 10 through 41.

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167, 169-182.

H. Gysin and E. Knusli, U.S. Patent 2,891,855, June 23, 1959, Col. 3, line 66 through Col. 5, line 17 and Examples 1-4.

G. C. Klingman, "Weed Control as a Science", John Wiley & Sons, Inc., New York, 1961, pp. 81-96.

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

EXAMPLE 4

Wettable Powder

| | |
|---|---|
| Methyl N'-(2,5-dimethoxyphenylsulfonyl)-N-(4,6-dimethoxypyrimidin-2-yl)carbamimidothioate | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until almost all of the solid particles thereof are under 50 microns in size, and then the ingredients are reblended.

EXAMPLE 5

Wettable Powder

| | |
|---|---|
| Methyl N'-(2,5-dimethoxyphenylsulfonyl)-N-(4,6-dimethoxypyrimidin-2-yl)carbamimidothioate | 50% |
| sodium alkylnapthalenesulfonate | 2% |
| low viscosity methyl cellulose | 2% |
| diatomaceous earth | 46% |

The ingredients are blended, coarsely hammermilled and then air-milled to produce particles

resulting suspension may be applied directly, but preferably after being extended with oils or emulsified in water.

## EXAMPLE 9

Wettable Powder

| | |
|---|---|
| Methyl N'-(2-chlorophenylsulfonyl)-N-(4,6-dimethoxypyrimidin-2-yl)carbamimidothioate | 20% |
| sodium alkylnapthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles almost all of which are below 100 microns in size, the material is reblended and sifted through a U.S.S. No. 50 sieve (0.3 mm opening) and packaged.

## EXAMPLE 10

Low Strength Granule

| | |
|---|---|
| Methyl N'-(2,5-dimethoxyphenylsulfonyl)-N-(4,6-dimethoxypyrimidin-2-yl)carbamimidothioate | 1% |
| N,N-dimethylformamide | 9% |
| attapulgite granules (U.S.S. 20-40 mesh) | 90% |

The active ingredient is dissolved in the solvent and the solution is sprayed upon dedusted attapulgite granules in a double cone blender. After spraying of the solution has been completed, the blender is allowed to run for a short period and then the granules are packaged.

## EXAMPLE 11

Aqueous Suspension

| | |
|---|---|
| Methyl N'-(2-chlorophenylsulfonyl)-N-(4-methoxy-6-methylpyrimidin-2-yl)-carbamimidothioate | 40% |
| polyacrylic acid thickener | 0.3% |
| dodecylphenol polyethylene glycol ether | 0.5% |
| disodium phosphate | 1% |
| Monosodium phosphate | 0.5% |

EXAMPLE 14

High Strength Concentrate

| | |
|---|---|
| Methyl N'-(2-chlorophenylsulfonyl)-N-(4,6-dimethoxypyrimidin-2-yl)carbamimidothioate | 99% |
| silica aerogel | 0.5% |
| synthetic amorphous silica | 0.5% |

The ingredients are blended and ground in a hammer-mill to produce a material almost all of which passes a U.S.S. No. 50 screen (0.3 mm opening). The concentrate may be formulated further if necessary.

EXAMPLE 15

Wettable Powder

| | |
|---|---|
| Methyl N'-(2-chlorophenylsulfonyl)-N-(4-methoxy-6-methylpyrimidin-2-yl)-carbamimidothioate | 90% |
| dioctyl sodium sulfosuccinate | 0.1% |
| synthetic fine silica | 9.9% |

The ingredients are blended and ground in a hammer-mill to produce particles almost all of which are below 100 microns in size. The material is sifted through a U.S.S. No. 50 screen and then packaged.

EXAMPLE 16

Wettable Powder

| | |
|---|---|
| Methyl N'-(2-chlorophenylsulfonyl)-N-(4,6-dimethoxypyrimidin-2-yl)carbamimidothioate | 40% |
| sodium ligninsulfonate | 20% |
| montmorillonite clay | 40% |

The ingredients are thoroughly blended, coarsely hammer-milled and then air-milled to produce particles almost all of which are below 10 microns in size. The material is reblended and then packaged.

sand mill until the solid particles have been reduced to under about 5 microns in size. The resulting suspension may be applied directly, but preferably after being extended with oils or emulsified in water.

EXAMPLE 20

Aqueous Suspension

| | |
|---|---|
| Methyl N'-(2-chlorophenylsulfonyl)-N-(4,6-dimethoxypyrimidin-2-yl)carbamimidothioate | 40% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles almost all of which are under 5 microns in size.

EXAMPLE 21

Low Strength Granule

| | |
|---|---|
| Methyl N'-(2-chlorophenylsulfonyl)-N-(4-methoxy-6-methylpyrimidin-2-yl)-carbamimidothioate | 0.1% |
| attapulgite granules (U.S.S. 20-40 mesh) | 99.9% |

The active ingredient is dissolved in a solvent and the solution is sprayed upon dedusted attapulgite granules in a double-cone blender. After spraying of the solution has been completed, the material is warmed to evaporate the solvent. The material is allowed to cool and then packaged.

UTILITY

The compounds of the present invention are useful as herbicides. They may be applied either pre- or post-emergence for selective weed control in crops or for the control of undesired vegetation in noncrop areas, such as weeds and brush growing on

ester, ethyl-N-benzoyl-N-(3,4-dichlorophenyl)-2-aminopropionate, 1,2-dimethyl-3,5-diphenylpyrazolium methylsulfate, methyl 2-[4-(2,4-dichlorophenoxy)-phenoxy]propanoate, 4-amino-6-tert-butyl-3-(methylthio)-1,2,4-triazin-5(4H)-one, 3-(3,4-dichlorophenyl)-1-methoxy-1-methylurea, 3-isopropyl-1H-2,1,3-benzothiodiazin-(4)-3H-one 2,2-dioxide, α,α,α-trifluoro-2,6-dinitro-N,N-dipropyl-p-toluidine, 1,1'-dimethyl-4,4'-bipyridinium ion, monosodium methanearsonate, 2-chloro-2',6'-diethyl(methoxymethyl)acetanilide, and 1,1-dimethyl-3-(α,α,α-trifluoro-m-tolyl)-urea.

The activity of these compounds was discovered in greenhouse tests. The tests are described and the data resulting from them are shown below.

Ratings for compounds tested by Procedure A through C described hereinbelow are:

0 = no effect
& or 10 = maximum effect
B = burn
C = chlorosis or necrosis
D = defoliation
E = emergence inhibition
G = growth retardation
H = formative effects
U = unusual pigmentation
6F = delayed flowering

TEST PROCEDURE A

Seeds of crabgrass (*Digitaria* spp.), barnyardgrass (*Echinochloa crusgalli*), wild oats (*Avena fatua*), cassia (*Cassia tora*), morningglory (*Ipomoea* spp.), cocklebur (*Xanthium* spp.), sorghum, corn, soybean, rice, wheat and nutsedge tubers (*Cyperus rotundus*) were planted in a growth medium and treated preemergence with a nonphytotoxic solvent solution of the compounds set forth in Table VIII.

TABLE VIII

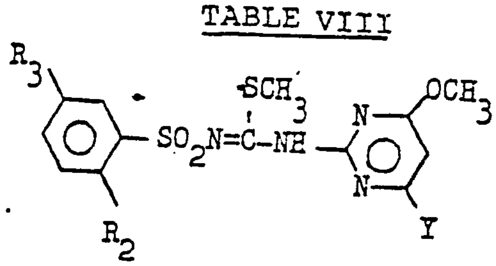

| $R_2$ | Cl | Cl | $CH_3O$ | $CH_3O$ |
|---|---|---|---|---|
| $R_3$ | H | H | $CH_3O$ | $CH_3O$ |
| Y | $CH_3$ | $CH_3O$ | $CH_3O$ | $CH_3O$ |
| kg/ha | 0.4 | 0.4 | 0.4 | 2.0 |
| POST EMERGENCE | | | | |
| BUSH BEAN | 3C,3H,9G | 2C,7H | 5G | 2C,2H,7G |
| COTTON | 2C,3H,9G | 2C,3H,8G | 1C,2H | 2C,2H,5G |
| MORNING GLORY | 9G | 2C | 1C | 5G |
| COCKLEBUR | 2H,8G | 1C | 6F | 6F |
| CASSIA | 9C | 1C,6G | 2C,5G | 5G |
| NUTSEDGE | 0 | 5G | 4G | 6G |
| CRABGRASS | 1C,4G | 4G | 4G | 1C,5G |
| BARNYARD GRASS | 9C | 8H | 1C,8H | 2H,7G |
| WILD OATS | 7G | 2G | 0 | 2G |
| WHEAT | 8G | 0 | 0 | 2G |
| CORN | 2U,9G | 5G | 2H,8G | 8G |
| SOYBEAN | 2C,9G | 3H,8G | 1C | 5G |
| RICE | 2C,9G | 5G | 4G | 6G |
| SORGHUM | 3U,9G | 3G | 2H,7G | 8G |
| PRE EMERGENCE | | | | |
| MORNING GLORY | 8G | 5G | 5G | 5G |
| COCKLEBUR | 8G | 10E | 0 | 6G |
| CASSIA | 9G | 5G | 0 | 1C |
| NUTSEDGE | 5G | 5G | 0 | 0 |
| CRABGRASS | 2C,8G | 5G | 0 | 2G |
| BARNYARD GRASS | 9H | 4C | 0 | 2G |
| WILD OATS | 8G | 0 | 0 | 4G |
| WHEAT | 9H | 0 | 0 | 4G |
| CORN | 9G | 3C | 3G | 0 |
| SOYBEAN | 9H | 1C | 0 | 1C |
| RICE | 10E | 4G | 2G | 2G |
| SORGHUM | 9G | 2C | 0 | 0 |

TABLE VIII continued

| | | | |
|---|---|---|---|
| | 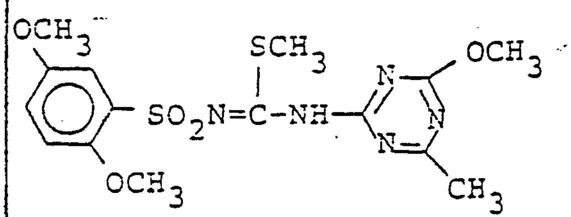 | | |
| kg/ha | 0.4 | | |
| POST-EMERGENCE | | | |
| Bush Bean | 3G | 9G | 6Y |
| Cotton | 4C | 9G | |
| Sorghum | 10C | 8G | |
| Corn | 2C | 6G | |
| Soybean | 2C | 7G | |
| Wheat | 2G | | |
| Wild Oats | 3C | | |
| Rice | 2C | 5G | |
| Barnyard Grass | 2C | 7H | |
| Crabgrass | 3G | | |
| Morningglory | 5C | 9G | |
| Cocklebur | 2C | | |
| Cassia | 2C | 7G | |
| Nutsedge | 2G | | |
| PRE-EMERGENCE | | | |
| Sorghum | 7G | | |
| Corn | 1C | 7G | |
| Soybean | 2C | | |
| Wheat | 5G | | |
| Wild Oats | 4G | | |
| Rice | 9H | | |
| Barnyard Grass | 7G | | |
| Crabgrass | 1C | | |
| Morningglory | 5G | | |
| Cocklebur | 2C | | |
| Cassia | 2C | 5G | |
| Nutsedge | 4G | | |

TABLE [illegible]

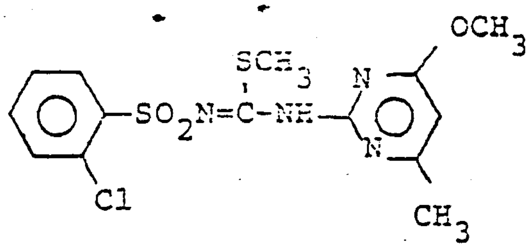

PRE-EMERGENCE HERBICIDE

Fallsington Silt Loam

| Rate, kg/ha | 1/4 | 1/16 |
|---|---|---|
| Crabgrass | 5G | 0 |
| Barnyardgrass | 9G,7H | 7G,7H |
| Sorghum | 9G,9C | 7G,5H |
| Wild Oats | 5C | 3G |
| Johnsongrass | 7G,5H | 6G |
| Dallisgrass | 5G,5C | 0 |
| Giant Foxtail | 9G,9C | 5G,3H |
| Ky. Bluegrass | 10E | 8G |
| Cheatgrass | 7G,5C | 3C,5G |
| Sugarbeets | 9G,9C | 3C,6G |
| Corn | 9G,9E | 5G,5H |
| Mustard | 10C | 8G |
| Cocklebur | 0 | 0 |
| Pigweed | 10C | 7G |
| Nutsedge | 10C | 0 |
| Morningglory | 5G | 0 |
| Cassia | 10C | 0 |
| Teaweed | 10C | 9C,6G |
| Velvetleaf | 10C | 5G |
| Jimsonweed | 7G,3C | 4G |
| Soybean | 5G,3H | 2H |
| Rice | 8G,8H | 8G,5H |
| Wheat | 6G | 0 |

0005986



TABLE X

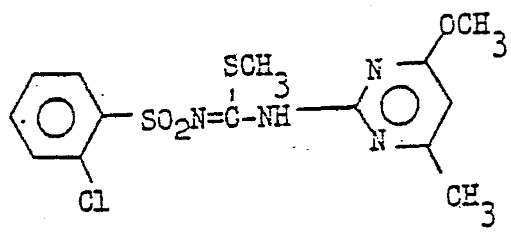

Over-the-Top Soil/Foliage Treatment

| Rate, kg/ha | 1/16 | 1/32 | 1/4 | 1/16 |
|---|---|---|---|---|
| Soybeans | 8G,5B | 5G | 10G,7C | 6C,10G |
| Velvetleaf | 10G,5C | 0 | 10G,7C | 8G,3H |
| Sesbania | 8G,7C | 0 | 10G,9C | 8G,4C |
| Cassia | 6G,2C | – | 10G,3C | 7G |
| Cotton | 7G,2C | 3G | 10G,4C | 8G,2C |
| Morningglory | 10G,8C | 0 | 10G,7C | 9G,5C |
| Alfalfa | 10G,8C | 3G | 10G,5C | 7G |
| Jimsonweed | 6G | 0 | 9G,3C | 9G,3C |
| Cocklebur | 2G | 0 | 4G | 4G |
| Corn | 10G | 7G,5H | 8G,2U | 8G,2U |
| Crabgrass | 0 | 0 | 2G | 0 |
| Rice | 7G,3C | 4G | 10G,2C | 10G |
| Nutsedge | 0 | 0 | 0 | 0 |
| Barnyardgrass | 8G,3H | 2G | 10G,2C | 10G |
| Wheat | 2G | 0 | 7G | 3G |
| Giant Foxtail | 4G | 2G | 10G | 8G |
| Wild Oats | 5G | 0 | 9G | 6G |
| Sorghum | 8G,3H | 5G | 10G | 10G |

2. A compound of Claim 1 wherein

$R_1$ is 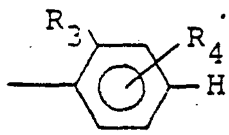

3. A compound of Claim 2 wherein
$R_3$ is Cl, Br, $CH_3$, $CH_3O$, $NO_2$ or $CO_2R_5$; and
$R_4$ is H or Cl.

4. A compound of Claim 3 wherein
$R_2$ is $C_1$-$C_6$ alkyl, $CH_2CH_2OCH_3$, $CH_2CH_2OC_2H_5$, $CH_2CH_2CH_2OCH_3$, $CH_2A$ or $-\overset{CH_3}{\overset{|}{C}}HA$; where A is $CO_2$ ($C_1$-$C_4$ alkyl), CN, $-CH=CH_2$ or $-C{\equiv}CH$.

5. A compound of Claim 4 wherein
$R^4$ is H; and
$R_3$ is Cl, $CH_3$, $NO_2$ or $CO_2R_5$.

6. A compound of Claim 5 wherein
$R_2$ is $C_1$-$C_4$ alkyl, $-CH_2A$ or $-\overset{CH_3}{\overset{|}{C}}HA$
where A is $CO_2CH_3$, $CO_2C_2H_5$, CN or $CH=CH_2$.

7. A compound of Claim 6 wherein
X is $CH_3$ or $OCH_3$.

8. The compound of Claim 2 or 7 wherein
$R_2$ is $CH_3$;
$R_3$ is Cl, $CH_3$ or $NO_2$; and
$R_4$ is H, Cl or $CH_3$.

9. Methyl N'-(2,5-dimethoxyphenylsulfonyl)-N-(4,6-dimethoxypyrimidin-2-yl)carbamimidothioate.

10. Methyl N'-(2-chlorophenylsulfonyl)-N-(4,6-dimethoxypyrimidin-2-yl)carbamimidothioate.

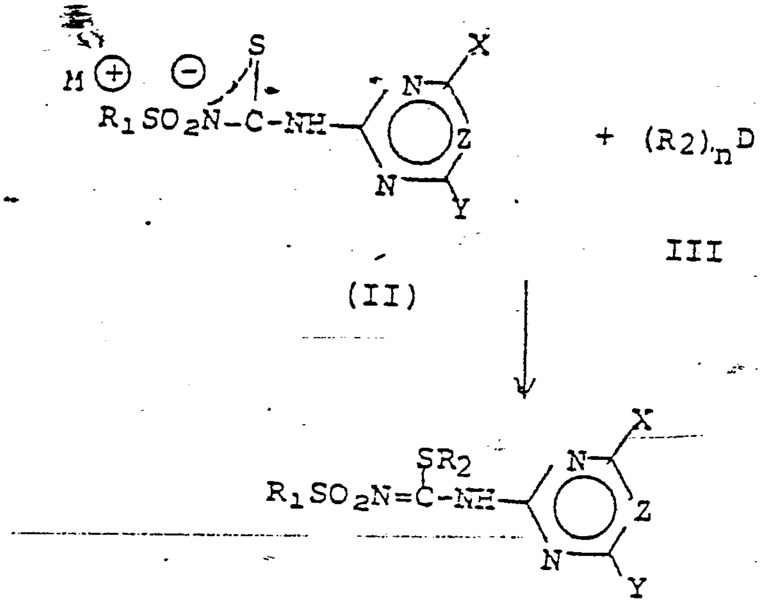

(II) + $(R2)_nD$ III

(I)

wherein $R_1$, $R_2$, X, Y and Z are as defined in Claim 1; D is $SO_4$, Cl, Br or I; M is an alkali metal or alkaline earth metal; and n is an integer corresponding to the valence of D; or

(b) contacting a compound of Formula IX with a compound of formula X:

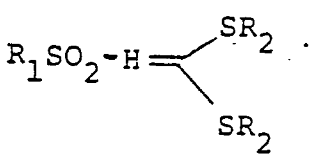

IX

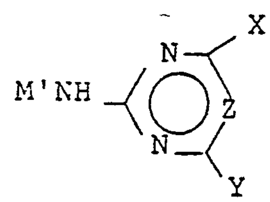

X

wherein $R_1$, $R_2$, X, Y and Z are as defined in claim 1 and M' is an alkali metal.

0005986

European Patent Office

# EUROPEAN SEARCH REPORT

Application number

79300976

| Category | Citation of document with indication, where appropriate, of relevant passages | Relevant to claim |
|---|---|---|
| | DE-A- 2 608 488 (Ciba-Geigy) + example 4 + | 18a |
| D,P | US-A- 4 127 405 (Levitt) + Columns 19 to 89 + | 15-17 |
| D | US-A- 3 714 209 (Tung & Powers) + Column 3,4 + | 16,17 |
| D | Chemical Abstracts vol 53 (1959) + 18052 g + | 18 |
| D | US-A- 3 637 366 (Wietelmann & Wittenbrook) + Columns 3 to 5 + | 15-17 |
| D | US-A- 3 823 007 (Stephens) + Columns 3 to 8 + | 15-17 |

----

CLASSIFICATION OF THE APPLICATION (Int. Cl.[3])

C 07 D 239/42
C 07 D 251/12
C 07 D 409/12
A 01 N 9/14
A 01 N 9/22
C 07 C 157/14

TECHNICAL FIELDS SEARCHED (Int.Cl.[3])

C 07 D 239/00, 02, 24, 28, 32, 42
C 07 D, 251/00, 02, 12
C 07 D 409/00, 02, 12
A 01 N 9/14
A 01 N 9/22
C 07 C 157/14

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| WIEN | 25 - 07 - 1979 | DASCHL |